# EUROPEAN PATENT SPECIFICATION

(11) **EP 1 353 628 B1**
(45) Date of publication and mention of the grant of the patent: **02.11.2006**
(21) Application number: 02700195.7
(22) Date of filing: 08.01.2002
(51) Int. Cl.: A61K 8/34, A61K 8/35, A61K 8/37, A61Q 5/00, A61Q 17/04

(54) **HAIR PROTECTION COMPOSITIONS**
HAARSCHUTZMITTEL
COMPOSITIONS DE PROTECTION CAPILLAIRE

(30) Priority: 16.01.2001 US 262055 P
(43) Date of publication of application: 22.10.2003
(73) Proprietor: DSM IP Assets B.V., 6411 TE Heerlen (NL)
(72) Inventor: DJERASSI, David, New York, NY 10022-8026 (US); MARKS, Alan, Martin, East Brunswick, NJ 08816 (US)
(74) Representative: Schwander, Kuno
(86) International application number: PCT/EP2002/000079
(87) International publication number: WO 2002/055029

(56) References cited:
- WO-A-00/62745
- WO-A-01/60330
- GB-A- 2 304 573
- US-A- 6 110 450
- DATABASE PROMT [Online] February 1995 (1995-02) "Burning questions" retrieved from STN Database accession no. 95:174921 XP002211636 & COMMUNITY PHARMACY, February 1995 (1995-02), page 6

## Description

The present invention relates to hair care compositions for protecting hair from the damaging effects of ultraviolet (UV) radiation, and hot blow-drying. More particularly, the invention relates to a hair care composition containing a UV-A filter, a UV-B filter, and phytantriol. Methods of making and using such compositions are also provided.

Tensile strength is one commonly accepted measure of the relative health of hair. Environmental insults, such as for example, UV radiation from exposure to sunlight and hot air blow drying, may damage hair, which can be determined by measuring the tensile strength hair.

It is known that hair passes through three phases while being extended under increasing force. The first phase, known as the elastic region, is characterized by reversible extension. Hydrogen bonds are thought to be the ones distributed in this region. The second phase, known as the yield region, is characterized by a partially reversible transformation in which covalent and ionic bonds are likely to be disturbed. Leading to the breaking point of a hair is the third region, also known as the post yield region.

It is also known that the breaking point of hair is related to hair diameter, and may not be an indication of overall hair damage. Thus, this parameter is not accurate or sensitive to measure hair damage. Because the yield region is the one most likely to correlate, in general, with covalent and possibly with disulfide bond breakage in hair, and because these are the types of bonds most notably affected by UV radiation, which results in intrinsic structural damage, evaluation of the yield slope may provide a practical and objective measurement of hair damaged by UV exposure.

Hot air blow drying may also damage hair, such as for example, by causing a reduction in tensile strength of the hair. The damage to the hair may be cumulative when the hair is sequentially exposed to UV radiation followed by hot air blow drying as occurs when one is exposed to the sun during the day and then washes, and blow dries the hair with a hot air blow drier in the evening. Such a sequence ofevents is typical when, for example, one is on vacation at the beach and is exposed to the sun during the day, and then in the evening washes and hot air blow dries the hair.

The present invention provides a hair care composition that includes a UV-A filter, a UV-B filter, and phytantriol. The composition contains from about 0.1% to about 0.5%(wt) of the UV-A filter, from about 0.1% to about 0.5%(wt) of the UV-B filter, and from about 0.1% to 0.5%(wt) of phytantriol. Preferably, to the composition contains about 0.2%(wt) of the UV-A filter, about 0.5%(wt) of the UV-B filter, and about 0.3%(wt) of phytantriol.

As used herein, the term "UV-A filter" means a compound or composition that absorbs UV radiation in the range of about 320 nm to about 400 nm. Such UV filters include, for example, butyl methoxydibenzoylmethane (PARSOL 1789), 4-(1,1-dimethylethyl)-4'-methoxydibenzoylmethane, 4-isopropyl dibenzoylmethane, 4-methoxy-benzylidene-cyanoacetic acid n-hexyl ester, 4-methoxy-benzylidene-cyanoacetic acid n-octyl ester, 4-methoxy-benzylidene-cyanoacetic acid n-decyl ester, 4-methoxy-benzylidene-cyanoacetic acid isononyl ester, 4-methoxy-benzylidene-ryanoacetic acid isodecyl ester, 4,4'-(6-(bis(2-ethyl-hexyl)-amino)-s-triazine-2,4-diyl)-diresorcinol, 2-(4-ethoxy-anilino-methylene)-propanedioic acid diethyl ester, terephtalylidene-3,3'-dicamphor-10,10'-disulfonic acid, 2-benzotriazol-2-yl-4-methyl-6-(3-(1,3,3,3-tetramethyl-1-(trimethyl silyl oxy)-disiloxanyl)-2-methyl-propyl)-phenol, 2,2'-(1,4-phenylen)-bis-1H-benzimidazol-4,6-disulfonic acid Na salt, hexyl 2-(4-diethylamino-2-hydroxy benzoyl) benzoate, ultrafine ZnO, and mixtures thereof. Preferably, the UV-A filter is selected from the group consisting of butyl methoxydibenzoylmethane, 4,4'-(6-(bis(2-ethyl-hexyl)-amino)-s-triazine-2,4-diyl)-diresorcinol, 2-(4-ethoxy-anilinomethylene)-propanedioic acid diethyl ester, and mixtures thereof. More preferably, the UV-A filter is butyl methoxydibenzoylmethane.

As used herein, the term "UV-B filter" means a compound or composition that absorbs UV radiation in the range of about 280 nm to about 320 nm. Such UV filters include, for example, octyl methoxycinnamate (PARSOL MCX), 2-ethylhexyl paramethoxycinnamate, oxybenzone, octyl salicylate, p-aminobenzoic acid, p-aminobenzoic acid ethyl ester oxyethylated in ethylene oxide, p-dimethylaminobenzoic acid ethyl ester, p-dimethylaminobenzoic acid amyl ester, p-dimethylaminobenzoic acid 2-ethylhexyl ester, p-dimethylaminobenzoic acid isoamyl ester, p-dimethylaminobenzoic acid hexyl ester, p-dimethylaminobenzoic acid heptyl ester, salicylic acid methyl ester, dipropylene glycol salicylate, salicylic acid homomenthyl ester, salicylic acid 2-ethylhexyl ester, triethanolamine salicylate, N-acetylanthranilic acid trimethylcyclohexyl ester, anthranilic acid menthyl ester, cinnamic acid benzyl ester, cinnamic acid menthyl ester and homomenthyl ester, cinnamic acid octyl ester, p-isopropylcinnamic acid ethyl ester, diisopropylcinnamic acid ethyl ester, diisopropylcinnamic acid methyl ester, p-methoxycinnamic acid isoamyl ester, p-methoxycinnamic acid isopropyl ester, p-methoxycinnamic acid propyl ester, p-methoxycinnamic acid 2-ethylhexyl ester, p-methoxycinnamic acid cyclohexyl ester, p-methoxycinnamic acid and salts, alpha-cyano-beta-phenylcinnamic acid ethyl ester, alpha-cyano-beta-phenylcinnamic acid 2-ethylhexyl ester, 2-hydroxy-4-methoxy-benzophenone, 4-phenylbenzophenone, 4-phenyl-benzophenone-2-carboxylic acid 2-ethylhexyl ester, 2,2'-dihydroxy-4-methoxy-benzophenone, 2-hydroxy-4-n-octyloxybenzophenone, 2,2'-dihydroxy-4,4'-dimethoxy-benzophenone, 2-hydroxy-4-methoxy-benzophenone-5-sulphonic acid, sodium 2,2'-dihydroxy-4,4'-dimethoxy-benzophenone-5-sulphonate, 2,4-dihydroxy-benzophenone,-2,2'-4,4'-tetrahydroxy-benzophenone, 2-hydroxy-4-methoxy-4'-methylbenzophenone, 3-(4-methylbenzylidene)-D,L-camphor, sodium 3-(4-methylbenzylidene)-D,L-camphor-sulphonate, 3-benzylidene-D,L-camphor, m-digallic acid trioleate, 2-phenylbenzimidazole-5-sulphonic acid, sodium 3,4-dimethoxyphenolglyoxylate, beta-imidazole-4(5)-acrylic acid (urocanic acid), 2-phenyl-5-methyl-benzoxazole, dibenzalazine, dianisoylmethyne, 5-(3,3-dimethyl-2-norbornylidene)-3-pent-2-en-2-one, (2'-hydroxy-5'-methylphenyl)-benzotriazole, 1-phenyl-3-(3-pyridyl)-1,3-propanedione, benzylidene malonate silicone polymer (PARSOL SLX), 2,4-Bis((4-(ethyl-hexylox)-2-hydroxy)-phenyl)-6-(4-methoxyphenyl)-1,3,5-triazine (Tinosorb S - Ciba), 2,2'-Methylene-bis-(6(2H-benzotriazol-2-yl)-4-(1,1,3,3,-tetramethylbutyl)phenol) (Tinosorb M - Ciba), Uvinul T-150 (BASF), UVASORB HEB (3V-Sigma), Ultrafine TiO₂, and mixtures thereof. Preferably, the UV-B filter is octyl methoxycinnamate or benzylidene malonate silicone polymer, more preferably, the UV-B filter is octyl methoxycinnamate.

In preferred compositions the UV-A filter is selected from the group consisting of PARSOL 1789, 4,4'-(6-(bis(2-ethyl-hexyl)-amino)-s-triazine-2,4-diyl)-diresorcinol (Triazin), 2-(4-ethoxy-anilinomethylene)-propanedioic acid diethyl ester, and mixtures thereof, and the UV-B filter is PARSOL MCX or PARSOL SLX.

Preferably, the hair care composition of the present invention contains PARSOL 1789, PARSOL MCX, and phytantriol.

The hair care composition may also include a cosmetically acceptable excipient. As used herein, the phrase "cosmetically acceptable excipient" means any compound or composition that is conventionally used to produce other desirable effects in hair care compositions. Thus, in the present invention, cosmetically acceptable excipients include, e.g., surfactants, buffering agents to adjust pH, agents to adjust viscosity, emollients, proteins, vitamins, minerals, fragrances, coloring agents, hair bleaches, fillers, and mixtures thereof. For example, in the present invention the cosmetically acceptable excipient may be lauramide DEA, polysorbate 80, sodium laureth sulfate, sodium chloride, citric acid, sodium EDTA, propylene glycol, cetyl alcohol, stearalkonium chloride, methylchloroisothiazolinone, methylisothiazolinone, water, or mixtures thereof.

Thus, the hair care composition containing a UV-A filter, a UV-B filter, and phytantriol may also contain lauramide DEA, polysorbate 80, sodium laureth sulfate, sodium chloride, citric acid, disodium EDTA, methylchloroisothiazolinone, methylisothiazolinone, and water. Alternatively, the hair care composition may include propylene glycol, cetyl alcohol, stearalkonium chloride, methylchloroisothiazolinone, methylisothiazolinone, and water.

An exemplary composition comprises about 0.2% (wt) of butyl methoxydibenzoylmethane; about 0.5% (wt) of octyl methoxycinnamate; about 0.3% (wt) of phytantriol; and a cosmetically acceptable excipient selected from the group consisting of lauramide DEA, polysorbate 80, sodium laureth sulfate, sodium chloride, citric acid, disodium EDTA, propylene glycol, cetyl alcohol, stearalkonium chloride; methylchloroisothiazolinone, methylisothiazolinone, water, and mixtures thereof.

In the present invention, the hair care composition may be incorporated into a variety of hair care products. Such hair care products include, for example, shampoos, hair conditioners, hair gels, hair mousses, hair sprays, and hair pomades. Preferably, the hair care composition is incorporated into a hair shampoo or a hair conditioner. As used herein, a "hair shampoo" is any composition that contains at least one surfactant suitable for cleaning hair, such as for example, the shampoo compositions set forth in the examples below.

The present invention also provides a process for producing a shampoo. This process includes combining a UV-A filter, a UV-B filter, phytantriol, an amide, and a non-ionic surfactant. In this process, the combining step may be achieved by mixing the components in any conventional mixing apparatus. An anionic surfactant is then mixed into the clear composition. Next, an aqueous solution containing a preservative is mixed into the composition. The pH of the mixture is then adjusted to between about 6.0 to about 7.2 with, e.g., a 50% (vol.) citric acid solution. The viscosity of the mixture is adjusted with, e.g., sodium chloride, and mixed until a uniform composition is formed.

Thus, another embodiment of the invention is a process for producing a shampoo comprising (a) mixing together a UV-A filter, a UV-B filter, phytantriol, an amide, and a non-ionic surfactant at about 65°C to about 70°C until a clear composition is formed; (b) mixing an anionic surfactant into the clear composition; (c) mixing an aqueous solution comprising a preservative into the composition of step (b) until the mixture is clear; (d) adjusting the pH of the mixture of step (c) to a pH of from 6.0 to 7.2 with a 50%(vol.) citric acid solution; and (e) adjusting the viscosity of the mixture of step (d) with sodium chloride, until a uniform composition is formed having at least a 80% protection value.

A hair shampoo may be produced using the process set forth above, wherein the shampoo includes 0.2%(wt) of a UV-A filter; 0.5%(wt) of a UV-B filter; 0.3%(wt) of phytantriol; 30%(wt) sodium laureth sulfate; 3.0%(wt) lauramide DEA; 0.10%(wt) disodium EDTA; 0.25%(wt) of a 50% solution of citric acid; 1.0%(wt) sodium chloride; 3.0%(wt) polysorbate 80; 0.05%(wt) of methylchloroisothiazolinone and methylisothiazolinone; and 61.6%(wt) water.

A hair shampoo comprising the composition of the invention imparts about 80% protection to the hair from the effects of sequential exposure to UV radiation followed by hot air blow drying.

In the present invention, a "hair conditioner" is any composition that is used to restore the original condition of hair. Such conditioners include, for example, silicones, cationic surfactants and quaternary ammonium compounds, and synthetic cationic polymers. Other components which have been described include moisturizing agents, thickeners or viscosity modifying agents for enhancing hand application, lathering agents for increasing foaming, foam stabilizers, and pearlizing agents. Examples of yet other components which are commonly included include perfumes, pH control agents, colorants, preservatives, and antimicrobials.

Other appropriate components for a hair conditioner may also be included in the hair conditioner composition. For example, in one aspect of this process, the UV-A filter, the UV-B filter, phytantriol, polypropylene glycol, cetyl acid, and stearalkonium chloride are mixed. Water is added with mixing, and the resulting mixture is heated. The mixture is cooled and an aqueous solution of a preservative is then added to the mixture. The mixture is allowed to cool to room temperature, with mixing, until a uniform composition is achieved.

Preferably, the UV-A filter is PARSOL 1789 and the UV-B filter is PARSOL MCX. In this process, the preservative is an aqueous solution containing methylchloroisothiazolinone and methylisothiazolinone. For this process, it is preferred to use the following compounds (in %wt): 0.2% of a UV-A filter; 0.5% of a UV-B filter; 0.3% ofphytantriol; 5.0% propylene glycol; 0.5% cetyl alcohol; 1.0% stearalkonium chloride; 0.05% of methylchloroisothiazolinone and methylisothiazolinone; and 90.45% water.

Thus, another embodiment of the invention is a process for producing a hair conditioner comprising (a) mixing together a UV-A filter, a UV-B filter, phytantriol, polypropylene glycol, cetyl acid, and stearalkonium chloride at about 75°C; (b) adding water at a temperature of about 65°C to the mixture of step (a) with mixing and heating until the mixture returns to a temperature of about 75°C; (c) cooling the mixture formed in step (b) to about 65°C; (d) adding a preservative to the mixture of step (c); and (e) allowing the mixture of step (d) to cool to room temperature with mixing until a uniform composition is formed.

Such a composition imparts about 80% protection from the effects of sequential exposure to UV radiation followed by hot air blow drying.

Another embodiment of the present invention is a process for protecting hair from the effects of sequential exposure to UV radiation followed by hot air blow drying. This process includes applying an effective amount of a composition containing from about 0.01% to about 2.5%(wt) of a UV-A filter, from about 0.01% to about 10%(wt) of a UV-B filter, and from about 0.01% to 2.5%(wt) of phytantriol to the hair prior to sequential exposure to UV radiation and hot air blow drying.

In the present invention, hair is "treated" with the compositions disclosed herein. Thus, the term "treated" means that the present compositions are applied to the hair using conventional methods, in sufficient quantities, and for a sufficient amount of time to achieve the desired protection. For example, in the case of a shampoo composition of the present invention, the hair is "treated" by applying an effective amount of the shampoo to the hair *e.g.,* manually or by spraying, etc. The shampoo is then worked into the hair so that substantially all of the hair is contacted with the shampoo. The shampoo is then rinsed out of the hair. As used herein, one application of, e.g., shampoo followed by a rinsing is designated as "one cycle." In the present invention, at least one cycle is required to treat the hair. Preferably, from 1 to 5 cycles may be used to treat the hair.

In this process, the composition preferably contains from about 0.1% to about 0.5%(wt) of the UV-A filter, from about 0.1% to about 0.5%(wt) of the UV-B filter, and from about 0.1% to about 0.5%(wt) of phytantriol. More preferably, the composition contains about 0.2%(wt) of the UV-A filter, about 0.5%(wt) of the UV-B filter, and about 0.3%(wt) of phytantriol.

In this process, it is preferred that the UV-A filter is PARSOL 1789, and that the UV-B filter is PARSOL MCX. It is also preferred that the amide is lauramide DEA, the non-ionic surfactant is polysorbate 80, and the anionic surfactant is sodium laureth sulfate. The aqueous solution may also contain disodium EDTA, methylchloroisothiazolinone, and methylisothiazolinone.

In the present invention, the compositions provide "protection" to the hair. As used herein, "protects," "protection," "protected," and "protecting" are used interchangeably to mean that the compositions disclosed herein reduce the damaging effects of UV radiation and hot air blow drying to the hair. Such damaging effects include reduction in the tensile strength of the hair.

The protection afforded to the hair by the compositions of the present invention is measured by the reduction in loss of tensile strength of hair treated with the present compositions prior to sequential exposure to UV radiation followed by hot air blow drying compared to untreated hair samples or samples treated with various other compositions.

In particular, percent protection ("% protection") is measured by dividing the tensile strength (in g/mm) of a hair sample that is untreated and not exposed to UV radiation or by hot air blow drying (control sample) by the tensile strength of a hair sample that is treated with a composition according to the present invention prior to sequential exposure to UV radiation followed by hot air blow drying (treated sample). Thus, % protection is provided by the following formula: (treated sample/control sample) * 100%.

In the present invention, "% protection values" are assigned to compositions that when used to treat hair provide a certain % protection as defined above. It is preferred that the present compositions have a % protection value of at least 80% or greater, more preferably of at least 88%.

Another embodiment of the invention is a method for reducing loss of tensile strength in hair caused by UV radiation and hot air blow drying. This method includes treating a hair with a composition containing about 0.01% (wt) to about 2.5% (wt) of a UV-A filter, about 0.01% (wt) to about 10% (wt) of a UV-B filter; and about 0.01% (wt) to about 2.5% (wt) of phytantriol, prior to exposure to UV radiation and hot air blow drying.

In this method the hair retains at least 80%, preferably at least 90 %, more preferably at least 95 % (conditioner) or at least 90 % (shampoo), of its tensile strength as compared to the control (untreated/unexposed hair).

The following examples are provided to further illustrate the compositions processes and methods of the present invention. These examples are illustrative only and are not intended to limit the scope of the invention in any way.

### Example 1: UV Radiation and Hot Air Blow Drying

Hair swatches (Virgin Brown hair) of approximately 20 cm in length were used in the following examples.

To simulate one week worth of exposure to UV radiation, hair swatches were placed about 66 cm from a Kratos Solar Simulator (Xenon arc lamp providing a highly intensified spectrum of light closely resembling that of the solar radiation) set at 20 V and 30 A of current for three hours. To simulate one weeks worth of blow drying hot air was blown on the hair at a distance of about 30 cm with a hair dryer at a high heat setting for 2 minutes.

The tensile strength of the hair was measured over the yield region. A single strand of hair 20 cm in length was extended with a force at a load setting of 20 g at a speed of 100 mm/min. For each treatment group or control, the tensile strength of 10 strands of hair were measured and the mean yield region slope was calculated.

**Table 1: Effect of sequential exposure of hair to UV radiation followed by hot air blow drying on the tensile strength of the hair.**

| | tensile strength [g/mm] | reduction in tensile strength [%] |
|---|---|---|
| untreated | 0.55 | |
| air-dried | 0.522 | 5.1 |
| blow-dried | 0.46 | 16.4 |
| UV-A/B | 0.351 | 36.2 |
| UVA/B & blow-dried | 0.309 | 43.8 |

### Example 2: Effect of Shampoo Formulations on tensile strength of hair

Shampoo formulations were made as set forth in Table 2 below:

**Table 2**

| ***ingredients*** | ***Inventive Shampoo*** | ***Phytantriol Only*** | ***UV-AB Only*** | ***Control*** |
|---|---|---|---|---|
| PARSOL MCX | 0.5% | ― | | ― |
| PARSOL 1789 | 0.2% | ― | | ― |
| phytantriol | 0.3% | 0.3 | ― | ― |
| sodium laureth sulfate | 30.0% | 30.0% | 30.0% | 30.0% |
| lauramide DEA | 3.0% | 3.0% | 3.0% | 3.0% |
| Versene Na2 | 0.1% | 0.1% | 0.1% | 0.1% |
| citric acid 50% | 0.25% | 0.25% | 0.25% | 0.25% |
| sodium chloride | 1.0% | 1.0% | 1.0% | 1.0% |
| Tween 80 | 3.0% | 3.0% | 3.0% | 3.0% |
| methylchloroisothiazolinone (and) methylisothiazolinone (Kathon CG) | 0.05% | 0.05% | 0.05% | 0.05% |
| deionized water | 61.6% | 62.3% | 61.9% | 62.6% |

Undamaged hair was treated with the respective shampoos set forth in Table 2 for 1 or 5 cycles. In each cycle, hair swatches were soaked in 25% shampoo solution for 2 min with constant agitation and then rinsed twice with warm water (40°C). The treated hair was then sequentially exposed to UV radiation followed by hot air blow drying as set forth in Example 1.

The tensile strength of the hair swatches were measured using the procedures set forth in Example 1, and the measurements are set forth in Table 3 below:

**Table 3: Effect on the tensile strength of hair treated with a hair shampoo composition prior to sequential exposure to UV radiation followed by hot air blow drying.**

| | Hair tensile strength [g/mm] | | Hair tensile strength versus untreated [%] | | Protection [%] | |
|---|---|---|---|---|---|---|
| | 1 cycle | 5 cycles | 1 cycle | 5 cycles | 1 cycle | 5 cycles |
| MCX/1789/Phytantriol | 0.498 | 0.513 | 79.2 | 88 | 92.32 | 95.20 |
| MCX & 1789 | 0.479 | 0.482 | 69.7 | 70.1 | 88.78 | 89.43 |
| Phytantriol | 0.444 | 0.458 | 52.2 | 57.5 | 82.31 | 84.91 |
| Control | 0.34 | 0.348 | | | 62.97 | 64.55 |
| Untreated | 0.539 | | | | | |

As Table 3 indicates, the Inventive Shampoo provided significantly better protection (% protection) to the hair from damage caused by UV exposure followed by hot air blow drying compared to controls in both the 1 and 5 cycle experiments. This is a substantial improvement over phytantriol alone or a combination of PARSOL MCX and PARSOL 1789 alone.

### Example 3: Effect of Conditioner Compositions on tensile strength of hair

**Table 4: Hair conditioner compositions**

| ***Ingredients*** | ***Inventive Conditioner*** | ***Phytantriol Only*** | ***UV-A*/*B Only*** | ***Control*** |
|---|---|---|---|---|
| PARSOL MCX | 0.5% | ― | 0.5% | ― |
| PARSOL 1789 | 0.2% | ― | 0.2% | ― |
| phytantriol | 0.3% | 0.3% | ― | ― |
| propylene glycol | 5.0% | 5.0% | 5.0% | 5.0% |
| cetyl alcohol | 2.5% | 2.5% | 2.5% | 2.5% |
| stearalkonium chloride | 1.0% | 1.0% | 1.0% | 1.0% |
| methylchloroisothiazolinone (and) methylisothiazolinone (Kathon CG) | 0.05% | 0.05% | 0.05% | 0.05% |
| Deionized Water | 90.45% | 91.15% | 90.75% | 91.45% |

Undamaged hair was treated with the respective hair conditioners for 1 or 5 cycles as set forth in Example 2. In each cycle, hair swatches were soaked in a 50% solution of one of the conditioner formulations set forth in Table 4 for 2 minutes, and then rinsed twice with warm water (40°C).

The treated hair was then sequentially exposed to UV radiation and hot air blow drying as set forth in Example 1. The tensile strength of the respective hair swatches were measured using the method set forth in Example 1. The data are presented in Table 5 below:

**Table 5: Tensile strengths of hair before and after sequential exposure to UV radiation followed by hot air blow drying and the effects of the application of various hair conditioner compositions**

| | Hair Tensile Strength (g/mm) | | Protection [%] | |
|---|---|---|---|---|
| | 1 cycle | 5 cycles | 1 cycle | 5 cycles |
| MCX/1789/Phytantriol | 0.518 | 0.528 | 96.64 | 98.40 |
| MCX & 1789 | 0.486 | 0.503 | 90.53 | 93.88 |
| Phytantriol | 0.452 | 0.469 | 84.32 | 87.45 |
| Control | 0.350 | 0.362 | 65.24 | 67.46 |
| Untreated | 0.536 | | | |

As the data in Table 5 indicate, the Inventive Conditioner provided significantly better protection (% protection) to the hair compared to controls in both the 1 and 5 cycle experiments.

## Claims

1. A hair care composition comprising about 0.1 % (wt) to about 0.5% (wt) of a UV-A filter; about 0.1 % (wt) to about 0.5% (wt) of a UV-B filter; and about 0.1 % (wt) to about 0.5% (wt) of phytantriol.

2. A composition according to claim 1 comprising about 0.2% (wt) of butyl methoxydibenzoylmethane; about 0.5% (wt) of octyl methoxycinnamate; about 0.3% (wt) of phytantriol; and a cosmetically acceptable excipient selected from the group consisting of lauramide DEA, polysorbate 80, sodium laureth sulfate, sodium chloride, citric acid, disodium EDTA, propylene glycol, cetyl alcohol, stearalkonium chloride, methylchloroisothiazolinone, methylisothiazolinone, water, and mixtures thereof.

3. The hair care composition according to claim 1, **characterized in that** it is a hair shampoo comprising by weight 0.2% of a UV-A filter; 0.5% of a UV-B filter; 0.3% of phytantriol; 30% sodium laureth sulfate; 3.0% lauramide DEA; 0.10% disodium EDTA; 0.25% of a 50% (wt) solution of citric acid; 1.0% sodium chloride; 3.0% polysorbate 80; 0.05% of methylchloroisothiazolinone and methylisothiazolinone; and 61.6% water.

4. A process for producing a shampoo comprising:
(a) mixing together a UV-A filter, a UV-B filter, phytantriol, an amide, and a non-ionic surfactant at about 65°C to about 70°C until a clear composition is formed;
(b) mixing an anionic surfactant into the clear composition;
(c) mixing an aqueous solution comprising a preservative into the composition of step (b) until the mixture is clear;
(d) adjusting the pH of the mixture of step (c) to a pH of from 6.0 to 7.2 with a 50%(vol.) citric acid solution; and
(e) adjusting the viscosity of the mixture of step (d) with sodium chloride, until a uniform composition is formed.

5. The hair care composition according to claim 1, **characterized in that** it is a hair conditioner comprising by weight 0.2% of a UV-A filter; 0.5% of a UV-B filter; 0.3% of phytantriol; 5.0% propylene glycol; 2.5% cetyl alcohol; 1.0% stearalkonium chloride; 0.05% of methylchloroisothiazolinone and methylisothiazolinone; and 90.45% water.

6. A process for producing a hair conditioner comprising:
(a) mixing together a UV-A filter, a UV-B filter, phytantriol, polypropylene glycol, cetyl acid, and stearalkonium chloride at about 75°C;
(b) adding water at a temperature of about 65°C to the mixture of step (a) with mixing and heating until the mixture returns to a temperature of about 75°C;
(c) cooling the mixture formed in step (b) to about 65°C;
(d) adding a preservative to the mixture of step (c); and
(e) allowing the mixture of step (d) to cool to room temperature with mixing until a uniform composition is formed.

7. A process for protecting hair from the effects of UV radiation and hot air blow drying comprising applying an effective amount of a composition comprising about 0.01 % (wt) to about 2.5% (wt) of a UV-A filter, about 0.01% (wt) to about 10.0% (wt) of a UV-B filter, and about 0.01 % (wt) to about 2.5% (wt) of phytantriol, to hair prior to exposure to UV radiation and hot air blow drying.

8. The use of a composition comprising about 0.01% (wt) to about 2.5% (wt) of a UV-A filter, about 0.01 % (wt) to about 10.0% (wt) of a UV-B filter, and about 0.01 % (wt) to about 2.5% (wt) of phytantriol for protecting hair from the effects of UV radiation and hot air blow drying comprising applying an effective amount of said composition to hair prior to exposure to UV radiation and hot air blow drying.

9. A method for reducing loss of tensile strength in hair caused by UV radiation and hot air blow drying comprising a treating step wherein a composition comprising about 0.01 % (wt) to about 2.5% (wt) of a UV-A filter, about 0.01 % (wt) to about 10% (wt) of a UV-B filter; and about 0.01% (wt) to about 2.5% (wt) of phytantriol is applied to hair.

## Patentansprüche

1. Haarpflegezusammensetzung, die ungefähr 0,1 (Gew.-)% bis ungefähr 0,5 (Gew.-)% UV-A-Filter, ungefähr 0,1 (Gew.-)% bis ungefähr 0,5 (Gew.-)% UV-B-Filter und ungefähr 0,1 (Gew.-)% bis ungefähr 0,5 (Gew.-)% Phytantriol enthält.

2. Zusammensetzung nach Anspruch 1, die ungefähr 0,2% (Gew.-)% Butylmethoxydibenzoylmethan; ungefähr 0,5% (Gew.-)% Octylmethoxycinnamat; ungefähr 0,3% (Gew.-)% Phytantriol; und einen kosmetisch akzeptablen Hilfsstoff ausgewählt aus der Reihe von Lauramid-DEA, Polysorbat 80, Natrium-Laureth-Sulfat, Natriumchlorid, Zitronensäure, Dinatrium-EDTA, Propylenglycol, Cetylalkohol, Stearalkoniumchlorid, Methylchlorisothiazolinon, Methylisothiazolinon, Wasser und deren Mischungen enthält.

3. Haarpflegezusammensetzung nach Anspruch 1, **dadurch gekennzeichnet, daß** es sich um ein Haarshampoo handelt, das 0,2% UV-A-Filter, 0,5% UV-B-Filter, 0,3% Phytantriol, 30% Natrium-Laureth-Sulfat, 3,0% Lauramid-DEA, 0,10% Dinatrium-EDTA, 0,25% einer 50 (gew.-)%igen Zitronensäurelösung, 1,0% Natriumchlorid, 3,0% Polysorbat 80, 0,05% Methylchlorisothiazolinon und Methylisothiazolinon und 61,6% Wasser enthält.

4. Verfahren zur Herstellung eines Shampoos, bei dem man
(a) ein UV-A-Filter, ein UV-B-Filter, Phytantriol, ein Amid und ein nichtionisches Tensid bei ungefähr 65°C bis ungefähr 70°C solange miteinander vermischt, bis eine klare Zusammensetzung entsteht;
(b) ein anionisches Tensid in die klare Zusammensetzung einmischt;
(c) eine wäßrige Lösung, die ein Konservierungsmittel enthält, so lange in die Zusammensetzung von Schritt (b) einmischt, bis die Mischung klar ist;
(d) den pH-Wert der Mischung von Schritt (c) mit einer 50 (vol.-)%igen Zitronensäurelösung auf einen pH-Wert von 6,0 bis 7,2 einstellt; und
(e) die Viskosität der Mischung von Schritt (d) mit Natriumchlorid solange einstellt, bis eine einheitliche Zusammensetzung entsteht.

5. Haarpflegezusammensetzung nach Anspruch 1, **dadurch gekennzeichnet, daß** es sich um eine Haarpflegespülung handelt, die 0,2 Gew.-% UV-A-Filter, 0,5% UV-B-Filter, 0,3% Phytantriol, 5,0% Propylenglycol, 2,5% Cetylalkohol, 1,0% Stearalkoniumchlorid, 0,05% Methylchlorisothiazolinon und Methylisothiazolinon und 90,45% Wasser enthält.

6. Verfahren zur Herstellung einer Haarpflegespülung, bei dem man
(a) ein UV-A-Filter, ein UV-B-Filter, Phytantriol, Propylenglycol, Cetylalkohol und Stearalkoniumchlorid bei ungefähr 75°C vermischt,
(b) die Mischung von Schritt (a) mit Wasser mit einer Temperatur von ungefähr 65°C versetzt, und zwar unter Mischen und Erhitzen, bis die Mischung wiederum eine Temperatur von ungefähr 75°C erreicht;
(c) die in Schritt (b) entstandene Mischung auf ungefähr 65°C abkühlt;
(d) die Mischung von Schritt (c) mit einem Konservierungsmittel versetzt; und
(e) die Mischung von Schritt (d) auf Raumtemperatur kommen läßt, wobei solange gemischt wird, bis eine einheitliche Zusammensetzung entsteht.

7. Verfahren zum Schützen von Haar gegen die Auswirkungen von UV-Strahlung und Heissluftfönen, bei dem man eine wirksame Menge einer Zusammensetzung, die ungefähr 0,01 (Gew.-)% bis ungefähr 2,5 (Gew.-)% UV-A-Filter, ungefähr 0,01 (Gew.-)% bis ungefähr 10,0 (Gew.-)% UV-B-Filter und ungefähr 0,01 (Gew.-)% bis ungefähr 2,5 (Gew.-)% Phytantriol enthält, auf das Haar aufträgt bevor dieses UV-Strahlung und Heissluftfönen ausgesetzt wird.

8. Verwendung einer Zusammensetzung, die ungefähr 0,01 (Gew.-)% bis ungefähr 2,5 (Gew.-)% UV-A-Filter, ungefähr 0,01 (Gew.-)% bis ungefähr 10,0 (Gew.-)% UV-B-Filter und ungefähr 0,01 (Gew.-)% bis ungefähr 2,5 (Gew.-)% Phytantriol enthält, zum Schützen von Haar gegen die Auswirkungen von UV-Strahlung und Heissluftfönen, wobei man eine wirksame Menge dieser Zusammensetzung auf das Haar aufbringt, bevor dieses UV-Strahlung und Heissluftfönen ausgesetzt wird.

9. Verfahren zur Verringerung des Verlustes der Zugfestigkeit im Haar aufgrund von UV-Strahlung und Heissluftfönen, bei dem ein Behandlungsschritt durchgeführt wird, in dem eine Zusammensetzung, die ungefähr 0,01 (Gew.-)% bis ungefähr 2,5 (Gew.-)% UV-A-Filter, ungefähr 0,01 (Gew.-)% bis ungefähr 10,0 (Gew.-)% UV-B-Filter und ungefähr 0,01 (Gew.-)% bis ungefähr 2,5 (Gew.-)% Phytantriol enthält, auf das Haar aufgetragen wird.

## Revendications

1. Composition de soin capillaire, comprenant d'environ 0,1% (en poids) à environ 0,5% (en poids) d'un filtre UV-A ; d'environ 0,1% (en poids) à environ 0,5% (en poids) d'un filtre UV-B ; et d'environ 0,1% (en poids) à environ 0,5% (en poids) de phytantriol.

2. Composition selon la revendication 1, comprenant environ 0,2% (en poids) de butyl méthoxydibenzoylméthane ; environ 0,5% (en poids) de méthoxycinnamate d'octyle ; environ 0,3% (en poids) de phytantriol ; et un excipient cosmétiquement acceptable choisi parmi le groupe constitué par le lauramide DEA, le polysorbate 80, le sodium laureth sulfate, le chlorure de sodium, l'acide citrique, l'EDTA disodique, le propylène glycol, l'alcool cétylique, le chlorure de stéaralkonium, la méthylchloroisothiazolinone, la méthylisothiazolinone, l'eau, et des mélanges de ceux-ci.

3. Composition de soin capillaire selon la revendication 1, **caractérisée en ce qu'**il s'agit d'un shampooing capillaire comprenant en poids 0,2% d'un filtre UV-A ; 0,5% d'un filtre UV-B ; 0,3% de phytantriol ; 30% de sodium laureth sulfate ; 3,0% de lauramide DEA ; 0,10% d'EDTA disodique ; 0,25% d'une solution à 50% (en poids) d'acide citrique ; 1,0% de chlorure de sodium ; 3,0% de polysorbate 80 ; 0,05% de méthylchloroisothiazolinone et de méthylisothiazolinone ; et 61,6% d'eau.

4. Procédé de production d'un shampooing, comprenant :
(a) le mélange d'un filtre UV-A, d'un filtre UV-B, de phytantriol, d'un amide, et d'un agent tensioactif non ionique à une température allant d'environ 65°C à environ 70°C jusqu'à la formation d'une composition limpide ;
(b) le mélange d'un agent tensioactif anionique dans la composition limpide ;
(c) le mélange d'une solution aqueuse comprenant un conservateur dans la composition de l'étape (b) jusqu'à ce que le mélange soit limpide ;
(d) l'ajustement du pH du mélange de l'étape (c) jusqu'à un pH allant de 6,0 à 7,2 par une solution à 50% (vol.) d'acide citrique ; et
(e) l'ajustement de la viscosité du mélange de l'étape
(d) par du chlorure de sodium, jusqu'à la formation d'une composition uniforme.

5. Composition de soin capillaire selon la revendication 1, **caractérisée en ce qu'**il s'agit d'un après-shampooing capillaire comprenant en poids 0,2% d'un filtre UV-A ; 0,5% d'un filtre UV-B ; 0,3% de phytantriol ; 5,0% de propylène glycol ; 2,5% d'alcool cétylique ; 1,0% de chlorure de stéaralkonium ; 0,05% de méthylchloroisothiazolinone et de méthylisothiazolinone ; et 90,45% d'eau.

6. Procédé de production d'un après-shampooing, comprenant :
(a) le mélange d'un filtre UV-A, d'un filtre UV-B, de phytantriol, de polypropylène glycol, d'alcool cétylique et de chlorure de stéaralkonium à une température d'environ 75°C ;
(b) l'addition d'eau à une température d'environ 65°C au mélange de l'étape (a) sous agitation et chauffage jusqu'à ce que le mélange revienne à une température d'environ 75°C ;
(c) le refroidissement du mélange formé dans l'étape (b) jusqu'à environ 65°C ;
(d) l'addition d'un conservateur au mélange de l'étape (c) ; et
(e) de laisser refroidir le mélange de l'étape (d) jusqu'à température ambiante sous agitation jusqu'à la formation d'une composition uniforme.

7. Procédé de protection des cheveux des effets du rayonnement UV et du séchage à l'air chaud, comprenant l'application d'une quantité efficace d'une composition comprenant d'environ 0,01% (en poids) à environ 2,5% (en poids) d'un filtre UV-A, d'environ 0,01% (en poids) à environ 10,0% (en poids) d'un filtre UV-B, et d'environ 0,01% (en poids) à environ 2,5% (en poids) de phytantriol, aux cheveux préalablement à une exposition a du rayonnement UV et du séchage à l'air chaud.

8. Utilisation d'une composition comprenant d'environ 0,01% (en poids) à environ 2,5% (en poids) d'un filtre UV-A, d'environ 0,01% (en poids) à environ 10,0% (en poids) d'un filtre UV-B, et d'environ 0,01% (en poids) à environ 2,5% (en poids) de phytantriol, destinée à protéger les cheveux des effets du rayonnement UV et du séchage à l'air chaud, comprenant l'application d'une quantité efficace de ladite composition aux cheveux préalablement à une exposition du rayonnement UV et du séchage à l'air chaud.

9. Méthode de réduction de la perte de résistance à la traction des cheveux provoquée par rayonnement UV et séchage à l'air chaud, comprenant une étape de traitement dans laquelle une composition comprenant d'environ 0,01% (en poids) à environ 2,5% (en poids) d'un filtre UV-A, d'environ 0,01% (en poids) à environ 10% (en poids) d'un filtre UV-B, et d'environ 0,01% (en poids) à environ 2,5% (en poids) de phytantriol, est appliquée aux cheveux.
